# DEMANDE DE BREVET EUROPEEN

(11) **EP 3 316 158 A1**
(43) Date de publication de la demande: **02.05.2018**
(21) Numéro de dépôt: 17192915.1
(22) Date de dépôt: 25.09.2017
(51) Int. Cl.: G06F 19/00, A61G 12/00, A61J 7/00, G07F 17/00

(54) **PROCÉDÉ DE DISTRIBUTION DE MÉDICAMENTS À UNE PLURALITÉ DE PATIENTS**

(30) Priorité: 28.10.2016 FR 1660546
(71) Demandeur: Distraimed, 31380 Montastruc-la-Conseillere (FR)
(72) Inventeur: FOUBET, Michel, 31240 Saint-Jean (FR); FOUBET, Olivier, 82710 Bressols (FR); FOUBET, Grégory, 31000 Toulouse (FR)
(74) Mandataire: Cabinet BARRE LAFORGUE & associés

(57) **Abrégé**

L'invention concerne un procédé de distribution de médicaments à une pluralité de patients dans lequel on utilise des piluliers (18) par temps de prise contenant chacun pour un patient différentes prises unitaires de médicaments pour un même et unique temps de prise mais pour différentes journées ; on regroupe les différents piluliers (18) en une pluralité de groupes de piluliers par temps de prise comprenant chacun, dans un réceptacle (22) commun, différents piluliers (18) pour différents patients pour un même temps de prise ; pour la distribution des médicaments, on choisit un réceptacle (22) pour un temps de prise correspondant à l'heure de distribution, et on délivre les médicaments aux patients des piluliers (18) de ce réceptacle (22).

## Description

L'invention concerne un procédé de distribution de médicaments à une pluralité de patients tels que des patients demeurant dans un même établissement, en particulier un établissement médico-social tel qu'une maison de retraite, une maison pour personnes handicapées, un établissement de cure ou de soins (par exemple de thalassothérapie), un sanatorium, un hôpital, une clinique...

On connaît déjà des procédés de distribution de médicaments à des patients dans lesquels on utilise des piluliers à usage unique (jetables) permettant d'emballer et de regrouper les différents médicaments par patient sur une période de temps prédéterminée, par exemple une semaine, selon une prescription médicale. Chaque pilulier est constitué d'un emballage d'un seul tenant comprenant une pluralité de logements d'emballage, chaque logement d'emballage contenant un ensemble, dit prise unitaire de médicaments, d'au moins un médicament destiné à être délivré à un seul et même patient à un moment donné de la journée, dit temps de prise. Ainsi, FR2913333 et FR2876024 décrivent un pilulier hebdomadaire présentant vingt-huit logements d'emballage répartis selon quatre colonnes de sept logements, chaque logement d'emballage d'une même colonne correspondant à l'un des temps de prise de la journée (matin, midi, soir, coucher), les différents logements d'emballage d'une même ligne correspondant aux différents temps de prise d'un même jour de la semaine. L'usage suppose qu'un même pilulier doit nécessairement être dédié à un seul et même patient, et ne peut donc comporter que des médicaments destinés à un seul et même patient.

Dans tous les procédés connus de distribution de médicaments à une pluralité de patients avec de tels piluliers à une heure d'une journée, dite heure de distribution, on choisit un pilulier correspondant à ce patient pour la période en cours et on distribue la prise unitaire de médicaments destinée à ce patient contenue dans le logement d'emballage de ce pilulier dont le temps de prise correspond à l'heure et au jour de distribution. Pour faciliter cette distribution, les piluliers sont soit suspendus à proximité immédiate des patients (par exemple un montant du lit du patient), soit rassemblés dans une pièce d'infirmerie dans laquelle une infirmière chargée de la tournée de distribution aux différents patients sépare les différentes cellules des différentes prises unitaires des différents patients, et les place dans un plateau qu'elle utilise lors de sa tournée pour distribuer les prises unitaires de médicaments aux différents patients successivement, soit encore transportés dans un chariot de distribution.

Ces procédés de distribution connus présentent plusieurs inconvénients.

Tout d'abord, il arrive souvent que la prescription de certains patients de la pluralité de patients concernée ne comporte pas les quatre temps de prise quotidiens et/ou ne comporte pas une prise unitaire de médicaments pour chaque jour de la semaine. En conséquence, pour ces patients, le pilulier hebdomadaire comportera un (ou plusieurs) logement(s) d'emballage vide(s) inutilisé(s). Dans certains cas, par exemple dans les maisons de retraite, cette situation peut se retrouver pour la grande majorité des patients. Il en résulte en particulier un gaspillage de matière important et un coût inutile.

US8123036 décrit un blister pilulier comprenant une rangée de sept compartiments, chaque rangée correspondant à un temps de prise pour un patient et étant dotée de moyens d'assemblage à une ou plusieurs rangées juxtaposées permettant de former des groupes de blisters par patient en cas de prescription quotidienne multiple. Ce dispositif est néanmoins complexe et coûteux en fabrication. Il impose des assemblages spécifiques à réaliser selon les prescriptions médicales de chaque patient, ce qui est relativement long et coûteux en main-d'oeuvre. Il n'est en outre pas exempt de risques dans la mesure où différentes rangées de différents patients peuvent être assemblées par erreur les unes aux autres, entraînant une erreur dans la délivrance de médicaments à des patients.

WO 99/61324 décrit un appareil de distribution automatique d'articles médicaux dans des bacs réceptacles individuels réutilisables, chaque bac réceptacle comportant au moins un secteur individuel. Ces bacs réceptacles peuvent être des bacs associés à chaque patient d'un hôpital, et sont alors remplis par un robot de remplissage immédiatement avant chaque temps de prise de chaque journée en médicaments préemballés dans des paquets chemisés contenant chacun une dose d'un médicament. Un secteur de chaque bac contient les médicaments devant être délivrés à un patient pour un temps de prise normal de la journée. Les autres secteurs peuvent être utilisés pour délivrer des médicaments aux patients entre les temps de prise normaux. Pour la distribution des médicaments aux différents patients, l'infirmière utilise un chariot mobile contenant un bac pour chaque patient. L'utilisation de tels bacs surdimensionnés par rapport aux besoins réels de distribution aux temps de prise normaux entraîne une complexité importante et des coûts qu'il convient d'éviter. En outre, le chariot utilisé pour la distribution contenant un bac pour chaque patient, y compris pour les patients pour lesquels aucune distribution de médicaments ne doit intervenir, est source d'erreur.

WO 2015/148375 décrit un module de transport sécurisé de médicaments contenant une ou plusieurs doses d'un ou plusieurs médicaments pour un patient unique. Ce module est utilisé par le pharmacien pour préparer les doses destinées à un patient, puis est utilisé sur le lieu d'administration des médicaments en étant chargé sur un chariot mobile de distribution. L'utilisation de tels modules de transport contenant une seule prise unitaire de médicaments pour un patient nécessite un traitement individuel de chaque prise unitaire en ce qui concerne le remplissage du module, son transport sur le lieu de distribution et son utilisation pour la distribution, et est particulièrement coûteuse.

L'invention vise donc à pallier ces inconvénients.

Elle vise donc en particulier à proposer un procédé de distribution de médicaments à une pluralité de patients, qui soit plus économique, évite les gaspillages d'emballages à usage unique, et n'impose pas des manipulations longues, coûteuses en main-d'oeuvre, et pouvant être sources d'erreurs dans la délivrance de médicaments à des patients.

Elle vise plus particulièrement à proposer un procédé de distribution qui soit simple, rapide, parfaitement fiable, et, par conception, limite la quantité d'emballages à usage unique à celle strictement nécessaire aux différents patients.

Elle vise également à proposer un tel procédé de distribution qui soit compatible avec les habitudes des personnels de soins chargés de la distribution des médicaments aux patients dans les établissements, et avec les contraintes et réglementations en vigueur dans ces établissements et/ou pour la distribution de médicaments.

Pour ce faire, l'invention concerne un procédé de distribution de médicaments à une pluralité de patients dans lequel, pour chaque patient de ladite pluralité de patients, des médicaments destinés à ce patient étant emballés dans au moins un emballage d'un seul tenant, dit pilulier, comprenant une pluralité de logements d'emballage, chaque logement d'emballage contenant un ensemble, dit prise unitaire de médicaments, d'au moins un médicament destiné à être délivré à ce patient à un moment donné de la journée, dit temps de prise :
- pour la distribution d'une prise unitaire de médicaments à un patient de ladite pluralité de patients, on choisit un pilulier correspondant à ce patient et on délivre la prise unitaire de médicaments destinée à ce patient contenue dans un logement d'emballage de ce pilulier,
caractérisé en ce que :
- on utilise des piluliers, dits piluliers par temps de prise, contenant chacun, pour un patient de ladite pluralité de patients, différentes prises unitaires de médicaments pour un même et unique temps de prise mais pour différentes journées, chaque logement d'emballage d'un pilulier par temps de prise contenant une prise unitaire de médicaments pour ce même et unique temps de prise pour ce patient,
- on regroupe les différents piluliers par temps de prise de ladite pluralité de patients en une pluralité de groupes de piluliers par temps de prise, chaque groupe de piluliers par temps de prise comprenant, dans un réceptacle commun, différents piluliers par temps de prise pour différents patients de ladite pluralité de patients, tous les piluliers par temps de prise d'un même groupe de piluliers par temps de prise correspondant tous à un même et unique temps de prise,
- pour la distribution des médicaments à des patients de ladite pluralité de patients à une heure d'une journée, dite heure de distribution, on choisit un réceptacle contenant un groupe de piluliers par temps de prise pour un temps de prise correspondant à l'heure de distribution et à ces patients, et on délivre les prises unitaires de médicaments à ces différents patients en utilisant les piluliers par temps de prise de ce réceptacle.

Pour faciliter la distribution et/ou renforcer sa sécurité, avantageusement et selon l'invention, on utilise des piluliers par temps de prise dotés chacun de moyens d'identification du temps de prise de ce pilulier par temps de prise. Dans certains modes de réalisation possibles, c'est chaque logement d'emballage d'un pilulier par temps de prise qui porte de tels moyens d'identification du temps de prise de ce pilulier par temps de prise.

Par exemple, dans certains modes de réalisation avantageux et selon l'invention lesdits moyens d'identification du temps de prise comprennent au moins un code à lecture sans contact -notamment à lecture optique ou RFID-. Dans ces modes de réalisation, avantageusement un procédé selon l'invention est aussi caractérisé en ce que lors de la distribution d'une prise unitaire de médicaments à un patient on utilise un dispositif informatique doté d'au moins un lecteur de codes à lecture sans contact, et on lit le code à lecture sans contact identifiant le temps de prise du pilulier par temps de prise du patient à l'aide d'un tel lecteur, et en ce que le dispositif informatique est adapté pour comparer le temps de prise identifié par le code ainsi lu et émettre un signal d'alarme si ce temps de prise ne correspond pas au temps présent, c'est-à-dire à l'heure de distribution. Ainsi, la correspondance entre l'heure de distribution et le temps de prise de chaque pilulier par temps de prise est vérifiée et validée lors de la distribution à chaque patient.

De même, pour faciliter la distribution et/ou renforcer sa sécurité, avantageusement et selon l'invention, on utilise des piluliers par temps de prise dotés chacun de moyens d'identification du patient correspondant à ce pilulier par temps de prise. Dans certains modes de réalisation possibles, c'est chaque logement d'emballage d'un pilulier par temps de prise qui porte de tels moyens d'identification du patient.

Par exemple, dans certains modes de réalisation avantageux et selon l'invention lesdits moyens d'identification du patient comprennent au moins un code à lecture sans contact -notamment à lecture optique et/ou un circuit RFID-. Dans ces modes de réalisation, avantageusement un procédé selon l'invention est aussi caractérisé en ce qu'un code à lecture sans contact d'identification individuelle de chaque patient est associé à chaque patient, en ce que lors de la distribution d'une prise unitaire de médicaments à un patient on utilise un dispositif informatique doté d'au moins un lecteur de codes à lecture sans contact, on lit le code à lecture sans contact d'identification associé au patient à l'aide d'un tel lecteur, on lit le code à lecture sans contact d'identification du patient porté par le pilulier par temps de prise à l'aide d'un tel lecteur, et en ce que ledit dispositif informatique est adapté pour comparer les codes ainsi lus et émettre un signal d'alarme en cas de différence.

Rien n'empêche de prévoir qu'au moins un réceptacle contienne plusieurs groupes de piluliers correspondant respectivement à différents temps de prise. Néanmoins, de préférence, avantageusement et selon l'invention, chaque réceptacle contient un unique groupe de piluliers par temps de prise. Autrement dit, tous les piluliers d'un même réceptacle correspondent tous à un même temps de prise. Ainsi, pour un temps de prise correspondant à l'heure de distribution, on choisit un réceptacle correspondant à ce temps de prise contenant uniquement des piluliers pour ce temps de prise. De la sorte, la manipulation et la manutention des groupes de piluliers par temps de prise sont facilitées et fiabilisées, et la sécurité de la distribution des médicaments est d'autant plus améliorée.

Par ailleurs, dans certains modes de réalisation possibles de l'invention on utilise des réceptacles dotés chacun de moyens d'identification de chaque temps de prise des piluliers regroupés dans ce réceptacle. Ces moyens d'identification peuvent comprendre au moins un code à lecture sans contact -notamment à lecture optique et/ou un circuit RFID-. En variante ou en combinaison, des codes de couleur peuvent être utilisés pour identifier à la première visualisation chaque temps de prise associé à un réceptacle.

Quoi qu'il en soit, dans un procédé selon l'invention, des moyens d'identification du temps de prise de chaque pilulier par temps de prise sont apposés sur chaque pilulier par temps de prise et/ou sur chaque logement d'emballage de chaque pilulier par temps de prise et/ou sur chaque réceptacle contenant des piluliers par temps de prise correspondant à ce temps de prise.

En outre, avantageusement et selon l'invention, pour la distribution des médicaments à une heure de distribution, on transporte ledit réceptacle successivement auprès des différents patients pour lesquels ledit réceptacle contient un pilulier par temps de prise. Autrement dit, on effectue une tournée de distribution auprès des différents patients pour chaque heure de distribution en utilisant un réceptacle contenant des piluliers par temps de prise pour ces différents patients, le temps de prise des différents piluliers du réceptacle correspondant à l'heure de distribution.

Par exemple, dans certains modes de réalisation d'un procédé de distribution selon l'invention, on transporte ledit réceptacle sur un chariot doté d'un dispositif informatique, ledit réceptacle et le dispositif informatique étant adaptés pour coopérer l'un avec l'autre pour permettre l'identification dans ledit réceptacle d'un pilulier par temps de prise à utiliser pour un patient et l'enregistrement en mémoire de la distribution d'une prise unitaire de médicaments à un patient à partir d'un pilulier par temps de prise du réceptacle.

Par ailleurs, dans un procédé selon l'invention, les différents piluliers par temps de prise de ladite pluralité de patients peuvent être regroupés dans des réceptacles lors même de la fabrication des piluliers et sur un site de fabrication des piluliers (pharmacie) et/ou après leur transport dans un établissement. Quoi qu'il en soit, avantageusement et selon l'invention, on stocke les réceptacles des groupes de piluliers par temps de prise à proximité de ladite pluralité de patients.

Plus particulièrement, avantageusement et selon l'invention, ladite pluralité de patients demeurant dans un même établissement, on stocke les réceptacles des groupes de piluliers par temps de prise dans cet établissement. Selon les infrastructures immobilières de cet établissement et ladite pluralité de patients faisant l'objet d'un même procédé de distribution selon l'invention, différentes variantes peuvent être envisagées. Par exemple, lorsque les patients sont regroupés par étages dans un immeuble, il est possible de prévoir un lieu de stockage des différents réceptacles pour chaque étage. D'autres variantes sont possibles (stockage des réceptacles par couloir, par immeuble, par service, par secteur...).

Dans un procédé de distribution selon l'invention, rien n'empêche par principe d'utiliser des piluliers réutilisables. Néanmoins, avantageusement et selon l'invention on utilise des piluliers à usage unique. En particulier, avantageusement et selon l'invention chaque logement d'emballage d'un pilulier par temps de prise étant adapté pour pouvoir être séparé individuellement des autres logements d'emballage du pilulier par temps de prise, pour la distribution d'une prise unitaire de médicaments à un patient on sépare un logement d'emballage du pilulier par temps de prise de ce patient contenu dans ledit réceptacle.

Par exemple, on utilise des piluliers par temps de prise formés chacun d'un blister comprenant une rangée de cuvettes à couvercles amovibles (par exemple sous forme d'étiquettes), chaque cuvette formant un logement d'emballage du pilulier par temps de prise.

L'invention concerne plus particulièrement un tel procédé pour la distribution de médicaments sous forme galénique solide -notamment non intégralement pulvérulente, de préférence non pulvérulente- tels que des médicaments du type *Per os* reconditionnables par exemple des comprimés, des cachets, des gélules, des capsules, des pilules, des pastilles, des dragées... Elle s'étend également cependant à un procédé de distribution de médicaments pouvant présenter une autre forme galénique (par exemple au moins certains des médicaments étant sous forme liquide, par exemple des gouttes, des ampoules,...).

Ainsi, alors que les piluliers sont des emballages regroupant des médicaments de plusieurs prises unitaires de médicaments d'un même patient, et étaient donc traditionnellement systématiquement utilisés individuellement pour chaque patient séparément des autres piluliers destinés aux autres patients, l'inventeur a déterminé pour la première fois qu'il est en réalité possible et considérablement avantageux de regrouper des piluliers par temps de prise pour différents patients dans des réceptacles, chaque réceptacle correspondant à un temps de prise, et d'utiliser de tels réceptacles par temps de prise pour chaque distribution des médicaments. En effet, il en résulte que l'on évite d'utiliser des piluliers dont certains logements d'emballage restent vides, et que seule la quantité strictement nécessaire de piluliers et de logements d'emballage pour les différents patients est utilisée, chaque réceptacle pour un temps de prise ne comportant pas de pilulier pour chaque patient qui n'a pas de prescription pour ce temps de prise. Il en résulte également que la sécurité de la distribution est assurée, un patient pour lequel il n'existe pas de pilulier dans un réceptacle pour un temps de prise ne pouvant pas faire l'objet d'une distribution de médicaments par erreur pour une heure de distribution correspondant à ce temps de prise. Il est également à noter qu'un procédé selon l'invention reste parfaitement compatible avec les habitudes des personnels dans ce domaine technique, et ce aussi bien en ce qui concerne la fabrication des piluliers lors de l'emballage des médicaments par les pharmaciens, qu'en ce qui concerne la distribution des différents médicaments à l'aide de piluliers, notamment de piluliers à usage unique de faible coût.

L'invention concerne également un procédé de distribution de médicaments à une pluralité de patients caractérisé en combinaison par tout ou partie des caractéristiques mentionnées ci-dessus ou ci-après.

D'autres buts, caractéristiques et avantages de l'invention apparaîtront à la lecture de la description suivante donnée à titre non limitatif et qui se réfère aux figures annexées dans lesquelles :
- la figure 1 est un schéma synoptique fonctionnel d'un mode de réalisation d'un ensemble de dispositifs permettant la mise en oeuvre d'un procédé de distribution selon l'invention,
- la figure 2 est un schéma illustrant la distribution d'une prise unitaire de médicaments à un patient dans un procédé de distribution selon l'invention,

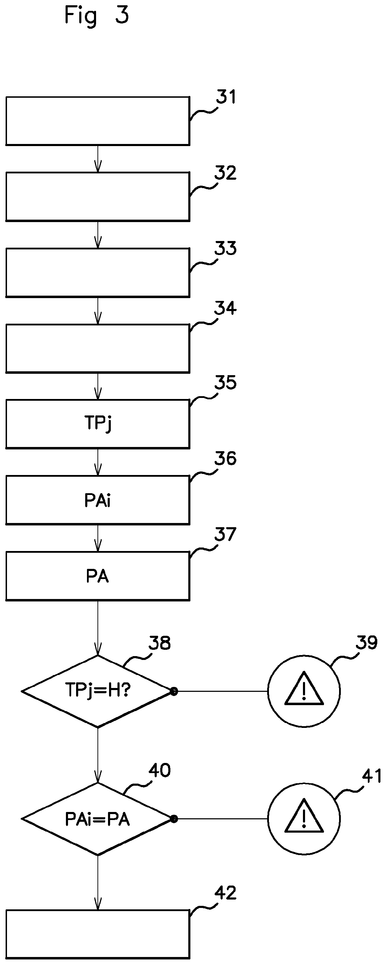

- la figure 3 est un logigramme illustrant un exemple de réalisation d'un procédé de distribution selon l'invention.

Dans l'exemple représenté, les dispositifs permettant la mise en oeuvre d'un procédé de distribution selon l'invention comprennent un premier dispositif 11 de préparation de piluliers sur un premier site, qui est par exemple une pharmacie, en fonction des prescriptions médicales de différents patients ; et un deuxième dispositif 12 pour la distribution des médicaments à une pluralité de patients situés sur un deuxième site, qui est par exemple un établissement dans lequel les patients demeurent.

Le premier et le deuxième site peuvent être distants l'un de l'autre comme représenté, les piluliers 18 préparés sur le premier site étant alors transportés, par exemple à l'aide de véhicules 13, sur le deuxième site. Rien n'empêche cependant bien sûr que les deux sites soient situés à proximité immédiate l'un de l'autre ou même qu'ils ne soient constitués que d'un seul site, les piluliers étant préparés dans l'établissement recevant les patients.

Sur le premier site, lors d'une première étape 31 d'un procédé selon l'invention, un pharmacien 14 prépare des piluliers 18 à partir de prescriptions médicales et de boîtes 15 de médicaments, par exemple à l'aide d'un dispositif d'assistance à la préparation de piluliers tel que décrit par exemple par FR2942132, relié à un dispositif 21 informatique permettant d'une part d'afficher les prescriptions médicales sur un écran 16 visible par le pharmacien, d'autre part d'enregistrer dans une base de données 17 des informations concernant les différents piluliers préparés, les différents médicaments utilisés et les différents patients. Cette base de données 17 permet ainsi d'assurer la traçabilité des opérations réalisées par le pharmacien 14.

L'enregistrement d'informations dans la base de données 17 par le pharmacien 14 peut être réalisé notamment à l'aide d'un lecteur 19 de codes à lecture sans contact (par exemple un lecteur optique de codes optiques tels que des codes à barres, ou un lecteur de codes à lecture par radiofréquence (RFID)) que le pharmacien 14 utilise pour lire des codes à lecture sans contact apposés sur les boîtes 15 de médicaments, et des codes à lecture sans contact imprimés sur des couvercles autocollants utilisés pour refermer les piluliers 18, couvercles qui sont imprimés par une imprimante 20 du dispositif 21 informatique après remplissage de chaque pilulier 18.

Dans un procédé selon l'invention, on utilise des piluliers 18 par temps de prise contenant chacun, pour un même et unique patient déterminé, différentes prises unitaires de médicaments pour un même et unique temps de prise, c'est-à-dire destinées à être délivrées à ce patient à un même et unique moment donné de la journée (par exemple matin, midi, soir, coucher ; ou autre), les différentes prises unitaires de médicaments contenues dans les différents logements 23 d'emballage de ce pilulier par temps de prise correspondant à différentes journées d'une période de référence de plusieurs jours de chaque pilulier.

Les piluliers 18 par temps de prise peuvent être des piluliers hebdomadaires comprenant sept logements 23 d'emballage correspondant aux sept jours de la semaine, chaque logement 23 d'emballage contenant une prise unitaire de médicaments pour le patient et pour ce temps de prise. Les piluliers 18 par temps de prise peuvent aussi être des piluliers bimensuels ou des piluliers mensuels, ou des piluliers bimestriels, ou des piluliers trimestriels ou autres, par exemple des piluliers pour des dates déterminées.

De préférence, les piluliers 18 par temps de prise sont des piluliers à usage unique (c'est-à-dire jetables après utilisation), par exemple sous forme de blisters transparents. Chaque logement 23 d'emballage d'un pilulier 18 peut être formé d'une alvéole transparente de blister refermée par un opercule amovible formé par le couvercle autocollant imprimé, et être ou non séparable des autres logements d'emballage du pilulier. Il peut être ou non compartimenté pour maintenir séparés les uns des autres les différents médicaments d'une même prise unitaire de médicaments.

Chaque logement 23 d'emballage d'un pilulier 18 par temps de prise porte avantageusement au moins un code 46 à lecture sans contact (code à lecture optique tel que code à barres et/ou un circuit RFID) identifiant dans la base de données 17 le patient, le temps de prise du pilulier 18, et la journée (de la semaine (lundi, mardi, mercredi, jeudi, vendredi, samedi ou dimanche), ou du mois (quantième), ou autre) de délivrance de la prise unitaire de médicaments de ce logement 23 d'emballage. Un même code à lecture sans contact peut être utilisé pour identifier l'ensemble de ces différentes informations, ou différents codes à lecture sans contact peuvent être utilisés pour identifier respectivement ces différentes informations.

Dans certains modes de réalisation, tous les piluliers 18 par temps de prise sont identiques, et présentent en particulier le même nombre de logements 23 d'emballage. En conséquence, il peut arriver que certains logements d'emballage d'un pilulier pour un patient particulier ne soient pas remplis, si ce patient ne doit pas recevoir une prise unitaire de médicaments tous les jours de la période de référence correspondant aux piluliers 18 par temps de prise.

Rien n'empêche cependant de prévoir en variante que chaque pilulier 18 par temps de prise présente au contraire un nombre de logements 23 d'emballage correspondant exactement au nombre de jours différents de la période de référence auquel le patient doit recevoir une prise unitaire de médicaments, par exemple si la période de référence correspond à la durée totale de traitement du patient. Dans cette variante, tous les logements 23 d'emballage d'un même pilulier 18 par temps de prise sont remplis nécessairement.

Il est également à noter que des codes de couleur peuvent être utilisés pour identifier à la première visualisation un temps de prise d'un pilulier 18 par temps de prise et/ou une journée de délivrance de la prise unitaire de médicaments d'un logement d'emballage d'un pilulier 18 par temps de prise.

Le pharmacien 14 prépare de préférence simultanément ou successivement les différents piluliers 18 par temps de prise nécessaires à un même patient pour les différents temps de prise de la période de référence des piluliers, avant de préparer les différents piluliers destinés à un autre patient.

Après la préparation de chaque pilulier 18, lors de l'étape 32 subséquente le pharmacien 14 place le pilulier 18 par temps de prise préparé dans un réceptacle 22 correspondant à ce temps de prise, c'est-à-dire permettant de regrouper les différents piluliers 18 par temps de prise correspondant tous à un même et unique temps de prise et destinés à différents patients d'une pluralité de patients pour lesquels une même tournée de distribution peut être effectuée dans un établissement.

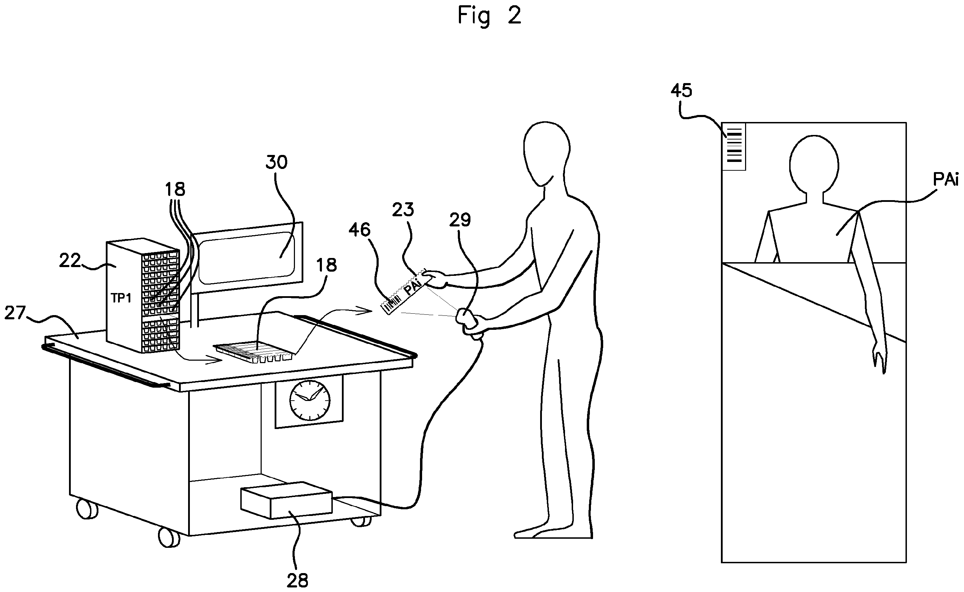

Dans l'exemple on a représenté quatre réceptacles 22 par temps de prise TPj, j variant de 1 à 4 : un premier réceptacle 22 correspondant à un premier temps de prise TP1 (par exemple matin) ; un deuxième réceptacle 22 correspondant à un deuxième temps de prise TP2 (par exemple midi) ; un troisième réceptacle 22 correspondant à un troisième temps de prise TP3 (par exemple soir) ; et un quatrième réceptacle 22 correspondant à quatrième temps de prise TP4 (par exemple coucher). Chaque réceptacle 22 est avantageusement formé d'une boîte telle que représentée figure 2 comprenant une pluralité d'étages de réception de blisters, chaque étage étant formé de rainures en regard permettant de recevoir entre elles un blister constituant un pilulier 18. Chaque réceptacle 22 permet donc de regrouper Nj différents piluliers 18 par temps de prise PPA1, PPA2..., PPAi,..., PPANj de Nj différents patients PA1, PA2..., PAi,..., PANj pour le temps de prise TPj au fur et à mesure de leur préparation par le pharmacien 14. Le nombre Nj de piluliers contenus dans chaque réceptacle 22 peut bien sûr varier d'un réceptacle à l'autre, tous les patients n'ayant pas nécessairement une prise de médicaments pour tous les temps de prise TPj des différents réceptacles 22. Au maximum, chaque réceptacle 22 comprend un nombre Nj de piluliers correspondant au nombre N de patients de ladite pluralité de patients pour lesquels une même tournée de distribution sera effectuée à l'aide des réceptacles 22.

Dans certains modes de réalisation avantageux, chaque réceptacle 22 présente des moyens d'identification de chaque temps de prise correspondant à ce réceptacle, par exemple sous la forme d'un code à lecture optique (code à barres par exemple) et/ou d'un circuit RFID et/ou d'un code de couleur permettant d'identifier chaque temps de prise des piluliers par temps de prise contenue dans ce réceptacle. Dans les modes de réalisation dans lesquels chaque réceptacle 22 contient des piluliers 18 par temps de prise correspondant tous à un même et unique temps de prise, ces moyens d'identification permettent d'identifier ce temps de prise unique. Par exemple, les réceptacles 22 peuvent être simplement de couleurs différentes en fonction du temps de prise correspondant. Néanmoins, dans un procédé selon l'invention chaque réceptacle 22 ne porte pas nécessairement un moyen d'identification de chaque temps de prise des piluliers contenus dans ce réceptacle.

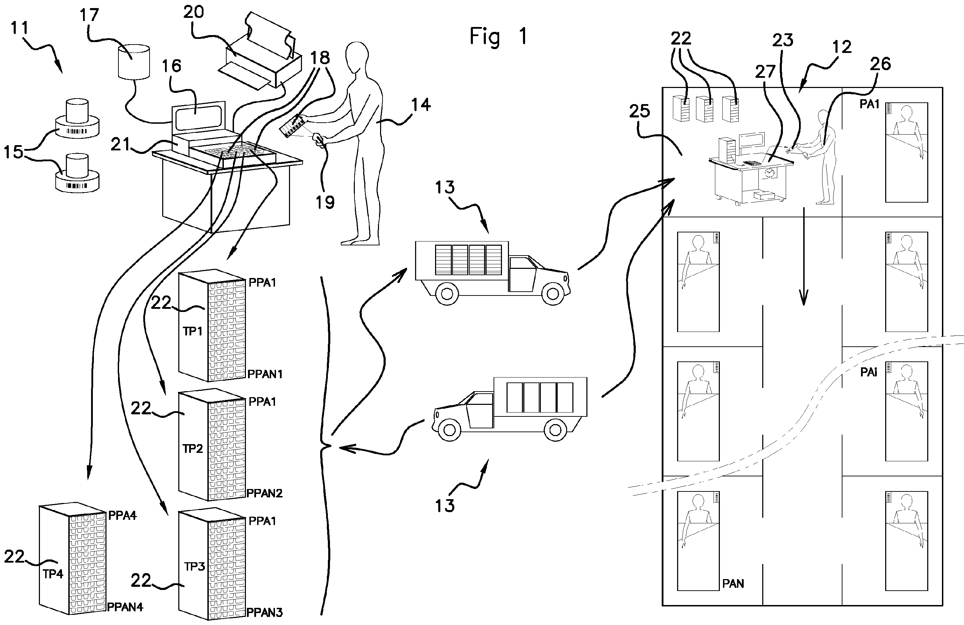

Une fois les piluliers 18 par temps de prise ainsi préparés pour chaque patient, puis regroupés dans les réceptacles 22 par temps de prise, ces réceptacles 22 sont transportés lors de l'étape 33 de transport vers le deuxième site, par exemple à l'aide de véhicules 13 de transport comme représenté schématiquement figure 1.

Dans l'exemple représenté, le deuxième site est un étage d'un établissement de soins logeant les N patients de ladite pluralité de patients. À cet étage, les différents réceptacles 22 par temps de prise livrés par un véhicule 13 sont stockés lors d'une étape 34 de stockage dans une salle 25, qui est par exemple une salle d'infirmerie. Éventuellement, les réceptacles 22 par temps de prise qui étaient présents dans la salle 25 mais ont été vidés après la distribution de tous les médicaments des piluliers qu'ils contenaient aux différents patients lors d'une période précédente, sont récupérés et transportés en retour sur le premier site pour être réutilisés pour stocker de nouveaux piluliers.

Pour réaliser une tournée de distribution de prises unitaires de médicaments aux différents patients à une heure de la journée, dite heure H de distribution, une infirmière 26 utilise un chariot roulant 27 permettant de transporter un réceptacle 22 et doté d'un dispositif 28 informatique comprenant notamment un lecteur 29 de codes à lecture sans contact et une interface 30 homme-machine comprenant notamment un écran et/ou un haut-parleur et/ou un clavier.

L'infirmière 26 choisit lors de l'étape 35 le réceptacle 22 contenant un groupe de piluliers 18 par temps de prise TPj pour un temps de prise correspondant à l'heure H de distribution et le place sur le chariot 27 qu'elle déplace ensuite successivement à proximité de chaque patient pour lui délivrer la prise unitaire de médicaments correspondant à ce temps de prise.

Lorsqu'elle arrive avec le chariot 27 à proximité d'un patient PAi, l'infirmière 26 utilise le lecteur 29 lors de l'étape 36 pour lire un code 45 d'identification du patient, porté par ce patient. Le dispositif informatique affiche alors sur l'écran l'identification de ce patient et la prescription correspondant à la prise unitaire de médicaments pour le temps de prise correspondant à l'heure H de distribution. En variante, il est possible que le dispositif 28 informatique soit adapté pour afficher la liste des patients, identifiés par leur état civil et/ou le numéro de chambre et/ou une photographie, pour lesquels des médicaments ont été prescrits et doivent être distribués pour ce temps de prise.

L'infirmière 26 choisit alors dans le réceptacle 22 le pilulier 18 correspondant à ce patient PAi, et utilise alors à nouveau le lecteur 29 lors de l'étape 37 pour lire chaque code 46 à lecture sans contact porté par le logement 23 d'emballage du pilulier 18 contenu dans le réceptacle 22 et destiné à ce patient.

Lors d'un test 38, le dispositif 28 informatique détermine tout d'abord si le temps de prise TPj du pilulier 18 par temps de prise choisi par l'infirmière tel que déterminé par un code 46 à lecture sans contact ainsi lu sur le logement 23 d'emballage du pilulier 18 correspond ou non à l'heure H de distribution et à la date en cours telles que déterminées par une horloge interne du dispositif 28 informatique. Par exemple, le temps de prise TPj correspond à l'heure H de distribution si cette heure H de distribution est comprise entre deux heures extrêmes de la journée définissant ce temps de prise. Par exemple, pour le temps de prise TP1 correspondant au matin, l'heure H de distribution doit être comprise entre 6 heures du matin et 11 heures. Le temps de prise correspond également à la date en cours si la journée de la période de référence du logement 23 d'emballage déterminé par le code 46 à lecture sans contact est identique à celle de la date en cours.

Dans la négative, un signal d'alarme est émis par le dispositif 28 informatique lors de l'étape 39, par exemple sur l'écran et/ou sur le haut-parleur pour prévenir l'infirmière 46 d'une erreur. Dans l'affirmative, le dispositif 28 informatique détermine ensuite lors du test 40 si l'identification PAi du patient déterminée par le code 45 d'identification du patient porté par ce dernier et préalablement lu correspond ou non à l'identification PA du patient telle que déterminée par un code 46 à lecture sans contact lu sur le pilulier 18. Dans la négative, un signal d'alarme est émis par le dispositif 28 informatique lors de l'étape 41, par exemple sur l'écran et/ou sur le haut-parleur pour prévenir l'infirmière 46 d'une erreur. Dans l'affirmative, un signal de validation de délivrance peut être communiqué à l'infirmière 46 par le dispositif 28 informatique (via l'écran et/ou le haut-parleur), puis l'infirmière 46 détache le logement 23 d'emballage du pilulier 18 et le remet au patient lors de l'étape 42 de délivrance de la prise unitaire de médicaments au patient. Il est à noter qu'après cette délivrance de la prise unitaire de médicaments aux patients le pilulier 18 par temps de prise de ce patient contient en général encore d'autres logements d'emballage contenant des prises unitaires de médicaments pour le même temps de prise mais pour des journées différentes ultérieures, si la période de référence des piluliers par temps de prise n'est pas totalement écoulée, c'est-à-dire si la journée de la date en cours n'est pas la dernière journée de la période de référence.

L'infirmière déplace ensuite le chariot 27 à proximité du prochain patient, et ce jusqu'à ce qu'une prise unitaire de médicaments ait été délivrée à tous les patients pour lesquels un pilulier est présent dans le réceptacle 22.

L'utilisation du dispositif 28 informatique pour la distribution des prises unitaires de médicaments permet d'éviter toute erreur de délivrance, tout oubli ou tout doublon de délivrance.

Il va de soi que ce dispositif 28 informatique peut être plus ou moins complexe, et peut même être utilisé pour assister l'infirmière dans la sélection du pilulier 18 correspondant à un patient, soit parce que l'emplacement du pilulier dans le réceptacle 22 a été enregistré dans la base de données 17 lors de la préparation des piluliers 18 et des réceptacles 22 et que le dispositif 28 informatique accède également à cette base de données 17 et indique cet emplacement à l'infirmière sur l'écran, soit que le réceptacle 22 est lui-même connecté au dispositif 28 informatique et doté de moyens par exemple lumineux facilitant l'identification du pilulier 18 à utiliser après lecture du code 45 d'identification du patient par le lecteur 29.

L'invention peut faire l'objet de nombreuses variantes de réalisation et de diverses applications autres que celles décrites ci-dessus et représentées sur les figures. Les réceptacles 22 peuvent présenter une toute autre structure, être ou non complètement fermés, dotés d'un dispositif de verrouillage/déverrouillage, éventuellement dotés de mécanismes et/ou automatismes permettant de les ouvrir et/ou de les déverrouiller. Les piluliers 18 peuvent également présenter une autre structure qu'un blister transparent à usage unique : il peut s'agir d'emballages dotés de plusieurs alvéoles à couvercles ou autres. Le procédé de distribution selon l'invention peut être mis en oeuvre dans diverses applications, non seulement dans le cadre d'un unique étage d'un établissement, mais également par exemple dans un immeuble, ou dans plusieurs immeubles voisins, ou même dans une salle dans laquelle les différents patients sont rassemblés. Les codes 45 d'identification des patients ne sont pas nécessairement portés par ces derniers, mais peuvent être apposés sur un lit du patient ou à proximité de la porte d'une chambre du patient ou autre. Les codes à lecture sans contact peuvent être des codes à lecture optique tels que des codes à barres, ou des codes à lecture par radiofréquence ou autres. Les réceptacles 22 peuvent être utilisés avec leur ouverture verticale comme représenté, ou au contraire avec leur ouverture horizontale vers le haut, éventuellement regroupés dans des tiroirs.

## Revendications

1. Procédé de distribution de médicaments à une pluralité de patients dans lequel, pour chaque patient de ladite pluralité de patients, des médicaments destinés à ce patient étant emballés dans au moins un emballage d'un seul tenant, dit pilulier, comprenant une pluralité de logements d'emballage, chaque logement d'emballage contenant un ensemble, dit prise unitaire de médicaments, d'au moins un médicament destiné à être délivré à ce patient à un moment donné de la journée, dit temps de prise :
- pour la distribution d'une prise unitaire de médicaments à un patient de ladite pluralité de patients, on choisit un pilulier correspondant à ce patient et on délivre la prise unitaire de médicaments destinée à ce patient contenue dans un logement d'emballage de ce pilulier,
**caractérisé en ce que** :
- on utilise des piluliers, dits piluliers (18) par temps de prise, contenant chacun, pour un patient de ladite pluralité de patients, différentes prises unitaires de médicaments pour un même et unique temps de prise mais pour différentes journées, chaque logement (23) d'emballage d'un pilulier (18) par temps de prise contenant une prise unitaire de médicaments pour ce même et unique temps de prise pour ce patient,
- on regroupe les différents piluliers (18) par temps de prise de ladite pluralité de patients en une pluralité de groupes de piluliers par temps de prise, chaque groupe de piluliers par temps de prise comprenant, dans un réceptacle (22) commun, différents piluliers (18) par temps de prise pour différents patients de ladite pluralité de patients, tous les piluliers par temps de prise d'un même groupe de piluliers par temps de prise correspondant tous à un même et unique temps de prise,
- pour la distribution des médicaments à des patients de ladite pluralité de patients à une heure d'une journée, dite heure de distribution, on choisit un réceptacle (22) contenant un groupe de piluliers (18) par temps de prise pour un temps de prise correspondant à l'heure de distribution et à ces patients, et on délivre les prises unitaires de médicaments à ces différents patients en utilisant les piluliers par temps de prise de ce réceptacle (22).

2. Procédé selon la revendication 1, **caractérisé en ce qu'**on utilise des piluliers (18) par temps de prise dotés chacun de moyens d'identification du temps de prise de ce pilulier par temps de prise.

3. Procédé selon la revendication 2 **caractérisé en ce que** lesdits moyens d'identification du temps de prise comprennent au moins un code (46) à lecture sans contact.

4. Procédé selon la revendication 3 **caractérisé en ce que** lors de la distribution d'une prise unitaire de médicaments à un patient on utilise un dispositif (28) informatique doté d'au moins un lecteur (29) de codes à lecture sans contact, et on lit le code (46) à lecture sans contact identifiant le temps de prise du pilulier (18) par temps de prise du patient à l'aide d'un tel lecteur (29), et **en ce que** le dispositif (28) informatique est adapté pour comparer le temps de prise identifié par le code ainsi lu et émettre un signal d'alarme si ce temps de prise ne correspond pas au temps présent.

5. Procédé selon l'une quelconque des revendications 1 à 4, **caractérisé en ce qu'**on utilise des piluliers (18) par temps de prise dotés chacun de moyens d'identification du patient correspondant à ce pilulier par temps de prise.

6. Procédé selon la revendication 5 **caractérisé en ce que** lesdits moyens d'identification du patient comprennent au moins un code (46) à lecture sans contact.

7. Procédé selon la revendication 6 **caractérisé en ce qu'**un code (45) à lecture sans contact d'identification individuelle de chaque patient est associé à chaque patient, **en ce que** lors de la distribution d'une prise unitaire de médicaments à un patient on utilise un dispositif (28) informatique doté d'au moins un lecteur (29) de codes à lecture sans contact, on lit le code (45) à lecture sans contact d'identification associé au patient à l'aide d'un tel lecteur (29), on lit le code (46) à lecture sans contact d'identification du patient porté par le pilulier (18) par temps de prise à l'aide d'un tel lecteur (29), et **en ce que** ledit dispositif (28) informatique est adapté pour comparer les codes ainsi lus et émettre un signal d'alarme en cas de différence.

8. Procédé selon l'une des revendications 1 à 7 **caractérisé en ce que** chaque réceptacle (22) contient un unique groupe de piluliers (18) par temps de prise.

9. Procédé selon l'une des revendications 1 à 8 **caractérisé en ce que** pour la distribution des médicaments, on transporte ledit réceptacle (22) successivement auprès des différents patients pour lesquels ledit réceptacle (22) contient un pilulier (18) par temps de prise.

10. Procédé selon la revendication 9 **caractérisé en ce qu'**on transporte ledit réceptacle (22) sur un chariot (27) doté d'un dispositif (28) informatique, ledit réceptacle (22) et le dispositif (28) informatique étant adaptés pour coopérer l'un avec l'autre pour permettre l'identification dans ledit réceptacle d'un pilulier (18) par temps de prise à utiliser pour un patient et l'enregistrement en mémoire de la distribution d'une prise unitaire de médicaments à un patient à partir d'un pilulier (18) par temps de prise du réceptacle.

11. Procédé selon l'une des revendications 1 à 10 **caractérisé en ce qu'**on stocke les réceptacles (22) des groupes de piluliers par temps de prise à proximité de ladite pluralité de patients.

12. Procédé selon l'une des revendications 1 à 11 **caractérisé en ce que** ladite pluralité de patients demeurant dans un même établissement, on stocke les réceptacles (22) des groupes de piluliers par temps de prise dans cet établissement.

13. Procédé selon l'une des revendications 1 à 12 **caractérisé en ce que** chaque logement (23) d'emballage d'un pilulier par temps de prise étant adapté pour pouvoir être séparé individuellement des autres logements (23) d'emballage du pilulier (18) par temps de prise, pour la distribution d'une prise unitaire de médicaments à un patient on sépare un logement (23) d'emballage du pilulier par temps de prise de ce patient contenu dans ledit réceptacle (22).

14. Procédé selon l'une des revendications 1 à 13 **caractérisé en ce qu'**on utilise des piluliers (18) par temps de prise formés chacun d'un blister comprenant une rangée de cuvettes à couvercles amovibles, chaque cuvette formant un logement (23) d'emballage du pilulier par temps de prise.

15. Procédé selon l'une quelconque des revendications 1 à 14 pour la distribution de médicaments sous forme galénique solide.
